# EUROPEAN PATENT SPECIFICATION

(11) **EP 4 384 246 B1**
(45) Date of publication and mention of the grant of the patent: **26.08.2026**
(21) Application number: 22754950.8
(22) Date of filing: 29.07.2022
(51) Int. Cl.: A61M 5/50, A61M 39/10, A61M 39/16, B65D 85/00

(54) **TAMPER-EVIDENT CLOSURE WITH A SECURING STRAP HAVING CONTROLLED BREAKING**
MANIPULATIONSVERSCHLUSS MIT EINEM SICHERUNGSBAND MIT KONTROLLIERTEM BRUCH
FERMETURE INVIOLABLE AVEC UNE SANGLE DE SECURITE A RUPTURE CONTROLEE

(30) Priority: 13.08.2021 US 202163232879 P
(43) Date of publication of application: 19.06.2024
(73) Proprietor: Solventum Intellectual Properties Company, Eagan, MN 55121 (US)
(72) Inventor: DOMBROWSKI, Alan R., Saint Paul, Minnesota 55133-3427 (US); TRONESS, Adam S., Saint Paul, Minnesota 55133-3427 (US); VOSSEN, Val, Saint Paul, Minnesota 55133-3427 (US); POWELL, Dennard J., Saint Paul, Minnesota 55133-3427 (US)
(74) Representative: Vossius & Partner Patentanwälte Rechtsanwälte mbB
(86) International application number: PCT/IB2022/057084
(87) International publication number: WO 2023/017354

(56) References cited:
- WO-A1-2021/024139
- US-A- 5 830 195
- US-B1- 10 722 680

## Description

### Technical Field

The present disclosure relates to a tamper-evident closure. In particular, the present disclosure relates to a tamper-evident closure for medical access devices, such as intravenous access points, with a securing strap.

### Background

Patients receiving medical care will commonly have a medical access device, such as a vascular access device, inserted to assist the healthcare provider in delivering medications to the patient. Some medical access devices are for allowing fluids to be removed from a patient, like a urinary catheter or a surgical drain line. When a person is under the care of a healthcare provider it is important to control the medications that are delivered to the patient. Patients with psychiatric conditions, intellectually delayed patients, pediatric patients, or intravenous drug users may tamper with intravenous access points or intentionally deliver drugs or medications to the vascular access device. Unprescribed drugs entering the vascular access device can cause medical problems, drug interactions, blood stream infections. WO 2021/024139 A1 discloses a tamper-evident closure which encloses a medical access device, such as a vascular access device, such that it will be visibly apparent if the medical access device contained in the tamper-evident closure becomes exposed. The tamper-evident closures comprise a holding cavity, a securing mechanism, and an apparent release. US 5 830 195 A discloses a medical coupling which has male and female members which push fit together, and a screw cap which secures the members against separation. To prevent unintentional release of the cap, locking means are provided between the cap and female member. US 10 722 680 B1 discloses an apparatus for detecting tampering which involves positioning at least a portion of one or more ingress/egress line having a first end and a second end in a clamping box configured to provide detection of tampering, and detecting tampering by observing the clamping box.

### Summary

The disclosed tamper-evident closure encloses a medical access device. It will be visibly apparent that the tamper-evident closure has been modified if the medical access device contained in the tamper-evident closure becomes accessed. The tamper-evident closure cannot be reapplied over the medical access device in a way to look the same as it did prior to exposing the medical access device.

The disclosed tamper-evident closure comprises a first part and a second part forming a holding cavity. The first part is connected with the second part such that actuation of the first part relative to the second part opens or closes access to the holding cavity. The closure further comprises an access point into the holding cavity and a securing strap comprising a first securing strap portion secured to the first part, a second securing strap portion with a free end for securing to the second part, and an area of weakness separating the first securing strap portion from the second securing strap portion. The force to break the area of weakness is less than the force to separate the securing strap from the first part or second part.

In one embodiment, the first part is pivotally connected to the second part.
In one embodiment, the tamper-evident closure further comprising a closure mechanism, wherein the closure mechanism comprises a tear strip that interlocks the first part with the second part along a perimeter of the holding cavity. In one embodiment, wherein the closure mechanism separates from the tamper-evident closure to release the interlock of the first part and second part.

In one embodiment, the securing strap includes a ramp edge aligned to the tear strip. In one embodiment, the area of weakness of the securing strip overlies the tear strip. In one embodiment, the area of weakness is a series of perforations.

In one embodiment, the first securing strap portion is integrally connected to the first part. In one embodiment, the second securing portion is irreversibly secured to the second part.

According to the invention, the second securing strap portion includes a latch extension and wherein the second part of the holding cavity includes a slot through the second part and a receiving latch to receive the latch extension. In one embodiment, the latch extension has one way engagement with the receiving latch, such that the latch extension will break and separate from the second securing strap with removal force. In one embodiment, the force to break the latch extension much greater than the force to separate the area of weakness on the strip. In one embodiment, a slot wall is within the holding cavity surrounding the latch extension. In one embodiment, the slot wall extends from the first part to the second part within the holding cavity.

In one embodiment, force to break the area of weakness is less than the force to break the latch extension connection with the slot.

In one embodiment, the first part includes an actuatable door for creating a second access point. In one embodiment, the closure further comprises a door support wall extending from the second part to engage with the actuatable door while the actuatable door is not actuated and is in connective engagement with the first part.

A method of enclosing a medical access device with tamper-evident closures comprises inserting the medical access device into the holding cavity and irreversibly securing the free end of the second securing strap to the second part. Removal of the closure mechanism breaks the securing strap.

### Brief Description of Drawings

FIG. 1 is a perspective view of one embodiment of a tamper-evident closure in an open position to expose the holding cavity and to receive a medical access device;
FIG. 2 is a perspective view of the tamper-evident closure of FIG. 1 in a closed position, with the securing strap retained and with a medical access device contained in the holding cavity;
FIG. 3 is a top view of the tamper-evident closure of FIG. 1 with the medical device;
FIG. 4 is a sectional view of the tamper-evident closure of FIG. 2 through the securing strap surrounding the holding cavity, with the medical access device removed;
FIG. 5 is a perspective view of the tamper-evident closure of FIG. 1 and 2 with the securing strap broken at the area of weakness to indicate tampering;
FIG. 6 is a perspective view of the tamper-evident closure of FIG. 1 and 2 where the tear strip has been released to open the tamper-evident closure and break the securing strap.

While the above-identified drawings and figures set forth embodiments of the invention, other embodiments are also contemplated, as noted in the discussion. In all cases, this disclosure presents the invention by way of representation and not limitation. Numerous other modifications and embodiments can be devised by those skilled in the art, which fall within the scope of this invention. The figures may not be drawn to scale.

### Detailed Description

Various designs for tamper-evident closures are disclosed. The disclosed tamper-evident closure 100 comprises a holding cavity 110, a securing strap 170, and optional closure mechanism 160 such that upon tampering or removal of the securing strap 170 there is a visually apparent indication that securing strap 170 had broken and has been released. The disclosed tamper-evident closures are well suited for securing a medical access device 200 for delivering fluid to a patient, removing fluid from a patient or connecting to other devices through a tube 220. In some embodiment, caps 230, such as a 3M^{™} Curos^{™} Disinfecting Cap that contain disinfectant are secured over the medical access device 200.

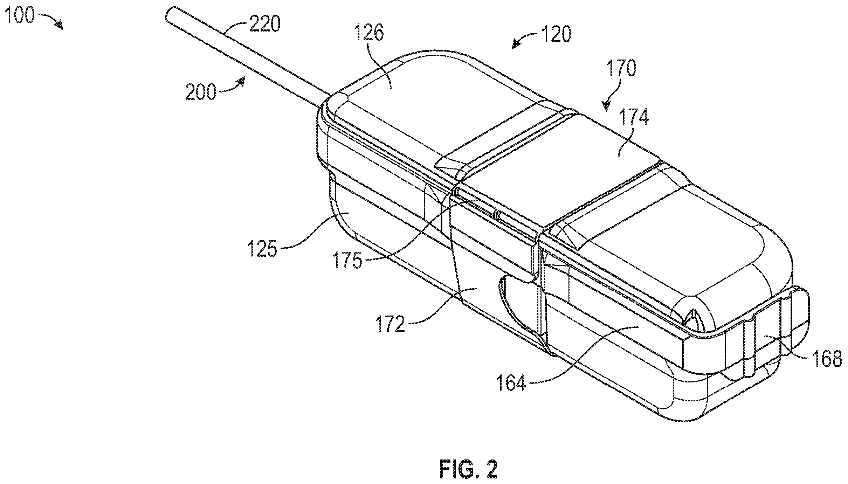

The holding cavity 110 has a length in the y direction, a width in the x direction, and a height in the z direction. The holding cavity has a perimeter 111 surrounding at least a portion of the holding cavity 110. At least one access point 113 is in the holding cavity 110, when the holding cavity is closed (FIG. 2, 5, 6) to allow for the tube 220 of the medical device to exit the holding cavity 110. Typically, the tamper evident closure 100 can be open so the access device 200 to be placed in the holding cavity 110 and closed to secure the access device 200 within the holding cavity 110.

In one embodiment the securing strap 170 retains the tamper-evident closure 100 closed. In one embodiment, an optional closure mechanism 160 retains the tamper-evident closure 100 closed. Various design of the holding cavity 110, securing straps 170, and optional closure mechanism 160 are contemplated and disclosed herein.

The holding cavity 110 of the tamper-evident closure 100 contains the medical access device 200. The medical access device 200 can be any site on a catheter or intravenous tubing where medications can be administered and/or where fluid can be removed. The medical access device 200 can be a vascular access device that is a connection to the patient's vascular system for either administering medication or removing blood. The medical access device 200 can be any portion of the infusion tubing, catheter, tube, or other medical device. This can be any site on a catheter, intravenous tubing, or other topical or percutaneous medical device. This may be a luer active device ("LAD") on a y-site, a LAD on the end of a line, or a male/female connection in the middle of the line or any other connection used on a topical or percutaneous medical device. A luer activated device can be either capped or un-capped.

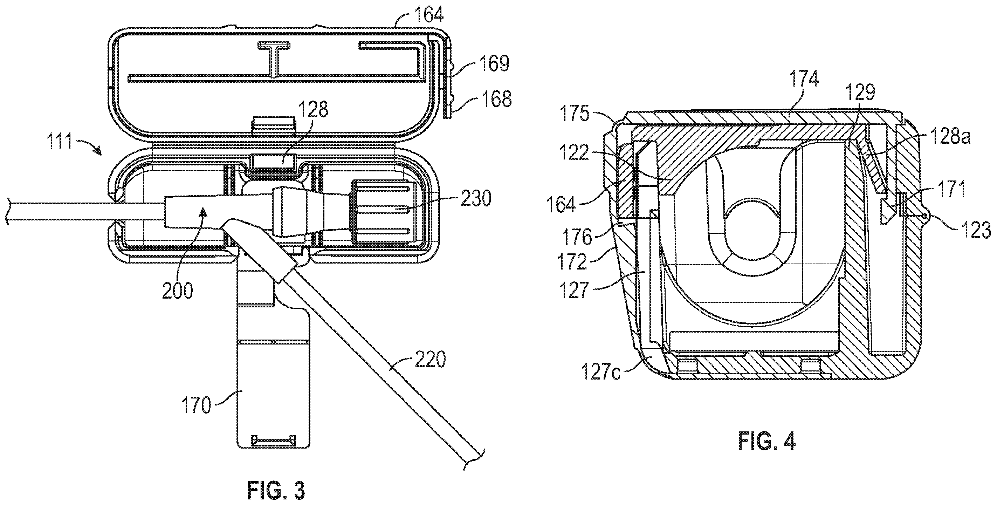

In some embodiments like shown in the figures, the fluid access point of the medical access device 200 is covered with a cap 230 (FIG. 3) for covering the fluid access point prior to use. Caps 230 can contain a disinfecting agent to keep the fluid access point 210 clean until use. Exemplary caps 230 are described in US Patents 7,282,186; 7,780,794; 9,907,617; 9,259,284. Exemplary caps 230 are sold as 3M^{™} CUROS^{™} Disinfecting Caps for Needleless Connectors, available from 3M Company.

When the holding cavity 110 is closed, the fluid access into the medical access device 200 is inaccessible to prevent access to the medical access device. If a user attempts to remove the securing strap 170 to open the tamper-evident closure, it will partially or completely separate and it will be visually apparent it has been tampered. If the tamper-evident closure 100 has been opened - either legitimately or through tampering - the securing strap 170 will completely separate and it will be visually apparent the tamper-evident closure 100 has been open.

In the disclosed tamper-evident closure 100, the securing strap 170 comprises an area of weakness 175. The force to break the area of weakness 175 is less than the force to separate the securing strap 170 from the tamper-evident closure 100. Therefore, even without opening the holding cavity 110, tampering will be evident from the broken area of weakness 170.

In some embodiment, materials that are highly resistant to breaking by cutting or impact, such as plastics, films, metals or ceramics, could be used to make the tamper-evident closure a tamper-resistant or even tamper-proof closure.

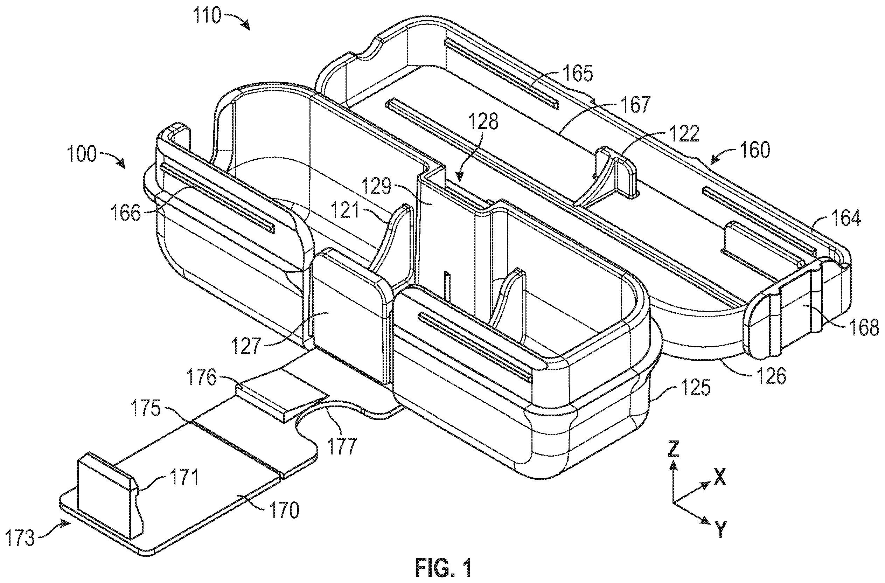

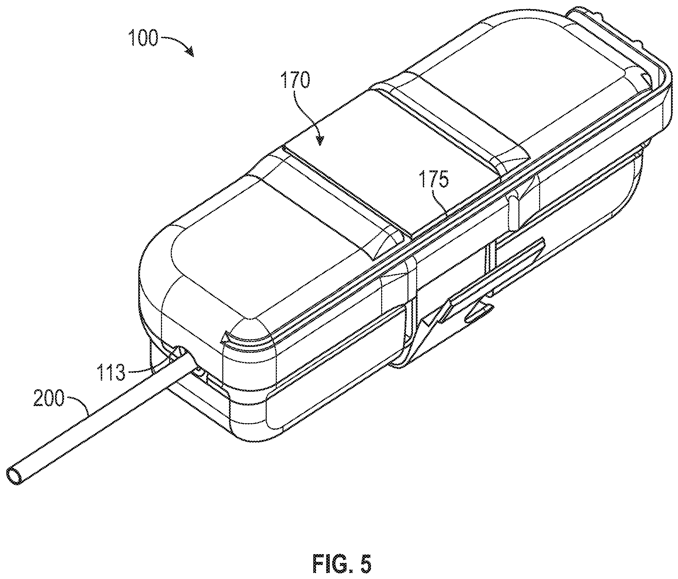

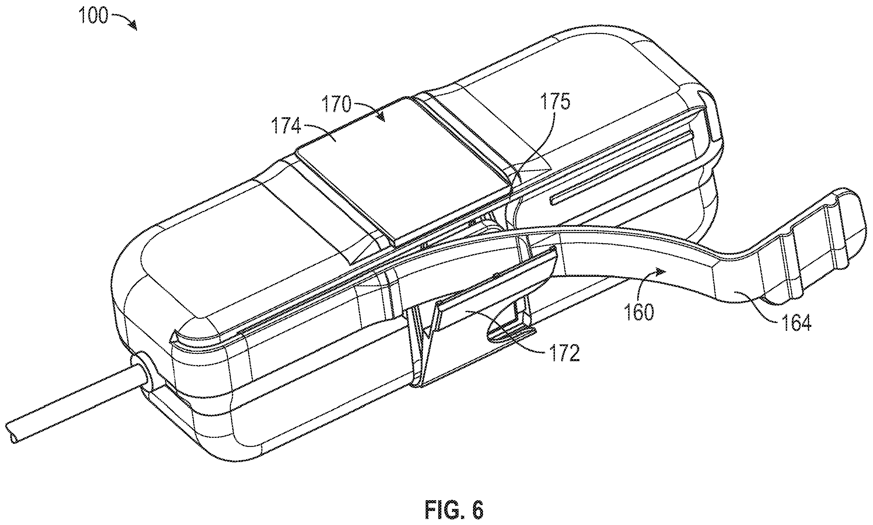

FIG. 1 is a perspective view of one embodiment of a tamper-evident closure 100 in an open position. FIG. 2 is a perspective view of the tamper-evident closure 100 in a closed with a medical access device 200 within the tamper-evident closure 100. FIG. 3 is a top view of the tamper-evident closure 100 of FIG. 1. FIG. 4 is a sectional view of the tamper-evident closure 100 of FIG. 2 though the securing strap 170 surrounding the holding cavity 110. FIG. 5 is a perspective view of the tamper-evident closure 100 with tampering of the securing strap 170 apparent such that the securing strap 170 broke at the area of weakness 175. FIG. 6 is a perspective view of the tamper-evident closure of FIG. 1 and 2 where the tear strip 164 of the closure mechanism 160, which was also holding the tamper-evident closure 100 closed, has been released to open the tamper-evident closure 100 and break the securing strap 175.

In this embodiment, the tamper-evident closure 100 comprises a first part 125 and a second part 126 that connect together to form the holding cavity 110. The medical access device 200 (FIGS. 2, 3, 5) can be placed in the first part 125, and then the second part 126 overlies the first part 125, like shown in FIG. 2. The first part 125 and second part 126 in an example that is not according to the invention, can be two separate parts but according to the invention, they are connected to one another by a hinge 123 (as best seen in FIG. 4). There are access points 113 in the tamper-evident closure 100 to allow the medical access devices tubing 220 to exit from the holding cavity 110 while the medical access device 200 is contained within the holding cavity 110. Other suitable designs of a tamper-evident closure 100 are shown and described in PCT Publication WO 2021/024139.

The securing strap 170 indicates tampering if a user was to attempt to remove the securing strap 170 from the tamper-evident closure 100 or to open the tamper-evident closure 100. In some embodiments, the securing strap 170 also functions to hold the tamper-evident closure 100 in a closed position. The securing strap 170 comprises a first securing strap portion 172 secured to the first part 125 and a second securing strap portion 174 that includes a free end 173 that is disconnected from the tamper-evident closure 100 before the tamper-evident closure 100 has been closed, like shown in FIG. 1 and 3. In one embodiment, the first securing strap portion 172 is rigidly, integrally, or substantially permanently connected with the first part 125. Between the first securing strap portion 172 and the second securing strap portion 172 is an area of weakness 175 of the securing strap 170. Various channels or protective areas of the tamper-evident closure are used to align the securing strap 170, prevent slipping or snagging of the securing strap 170 when the tamper-evident closure is being used.

As shown in FIG. 2, once the first part 125 closes over the second part 126, the securing strap 170 extends from the first part 125 to the second part 126. In this embodiment, the securing strap 170 extends around a substantial portion of the tamper-evident closure 100. In this embodiment, the securing strap 170 extend from one side of the tamper-evident closure 100 to the opposing side of the tamper evident closure 100, across the entire height (z-direction) of the tamper-evident closure 100.

Various mechanism could be included for attachment of the second securing strap portion 174 to the tamper-evident closure 100. For example, mechanical of interlocking mechanism, adhesive attachments of one or both ends, etc. As shown in the embodiment, the second securing strap portion 174 to the second part 126. Typically, the attachments is a one-way, irreversible connection of the second securing strap portion 174. For example, in one embodiment, the mechanical attachment to allow for the free end 173 to secure to the second part 126 and then for the free end 173 to release from the second part 126 can be included. In the embodiment shown, the second securing strap portion 174 includes a latch extension 171 for mechanical engagement within a receiving latch 128a which is contained within a slot 128 (see FIG. 3 and 4). The slot 128 passes through the second part 126 and within the slot 128 is the receiving latch 128a. In the embodiment shown, the latch extension 171 and receiving latch 128a interlock in a one-way, irreversible manner and therefore the interlock does not release. Here, the latch extension 171 will break if removal of the connection is attempted. This keeps the securing strap 170 securely connected to the first part 125 and second part 126. Also, in this embodiment, the securing strap 170 functions to hold the tamper-evident closure 100 in a closed position.

Optionally, within the holding cavity 110, the slot 128 for receiving the latch extension 171 is surrounded by a slot wall 129 that extends to prevent possible actuation of the latch extension 171 for release from the receiving latch 128a if tampering includes access from within the cavity pressing against the latch extension 171 (see FIG. 4). In other words, with an elongated tool, a user could press against the latch extension 171 and release it from the receiving latch 128a. The slot wall 129 is within the holding cavity 110. In the embodiment shown, the slot wall 129 extends from the first part 125 to the second part 126 and substantially surround the latch extension 171.

The latch extension 171 in this embodiment is a barb, but any number of other one-way mechanical interlocks are suitable. For example, the latch extension could be a protruding portion like a ball that inserts into a receiver.

The securing strap 170 includes an area of weakness 175 that enables controlled breaking when attempting to release the securing strap 170 or attempting to open the holding cavity 110. The area of weakness 175 could be any number of designs where the force to break the area of weakness 175 is less than the force to separate the securing strap 170 from the first part 125 or second part 126. In other words, attempted removal, opening, destruction of the securing strap 170 will break at the area of weakness 175 before the securing strap 170 connection of the latch extension 171 and slot 128 will break or release. For example, the area of weakness 175 can be a substantially thinner material than the other portions of the securing strap 170 or can be one or more perforations through the securing strap 170.

The slot 128 is shaped to be a container so that if the latch extension 171 broke from tampering, the latch extension 171 will separate from the free end 173 of the securing strap 170 and fall into the slot 128 and advantageously collects and contains the loose part of the broken latch extension 171.

In some embodiments, an adhesive, such as a permanent adhesive can be used as the mechanical attachment of the securing strap 170 to either the first part 125 or second part 126.

FIG. 5 depicts the tamper-evident closure 100 in a closed position but where a user has attempted to lift, remove, or release the second securing strap portion 172 from attachment with the second part 126. Here, the area of weakness 175 has separated the first securing strap portion 172 from the second securing strap portion 174 to indicate tampering. The area of weakness 175 broke before the latch extension 171 broke or released from the slot 128.

In some embodiment, the first part 125 and the second part 126 are held in a close position by an additional closure mechanism 160. In this embodiment, a closure mechanism 160 for holding the holding cavity 110 closed comprises a tear strip 164 extends around at least a portion of the perimeter of the tamper-evident closure 100 connecting the first part 125 and second part 126. The tear strip 164 comprises a mechanical interlocking system linking between the first part 125 and the second part 126. In the embodiment shown, the tear strip 164 comprises a longitudinally extending protruding portion 165 that interlocks with a longitudinally extending receiving portion 166 on the first part 125 for securing the tamper-evident closure 100 in a closed position. It is understood that the extending or receiving portions could be alternative be included on the other first part 126 or second part 126. The tear strip 164 has a line of weakness 167 adjacent to the tear strip 164 that connects the tear strip 164 to the second part 126 in this embodiment, but it is understood is could alternatively connect the tear strip 164 to the first part 125.

The tear strip 164 can have a tab 168 for a user to be able to easily grab the tear strip 164 to begin removal of the tear strip 164. Removal of the tear strip 164 separates the tear strip 164 from the second part 126 along the line of weakness 167. In this embodiment, the tab 168 is on one end of the tamper-evident closure 100. The tab 168 could be anywhere along the tear strip 164. Typically it is on an end of the tamper-evident closure 100 without an access point 113 so as not to interfere with the tube 220 that leaves the access point 113. However, if the tab 168 is on an end also containing an access point 113, the tab 168 is dimensioned and positioned to not interfere with the exiting tube 220. The tab 168 is therefore ideally positioned slightly removed from the tamper-evident closure 100, like shown in the top view of FIG. 3. However, to ensure the tab 168 is not so disconnected and to help strengthen the connection of the tear strip 164 to the closed holding cavity 110, optional tab ties 169 are included to secure a portion of the tab 168 to the tamper-evident closure 100. These tab ties 169 add strength to the tab 168 connection to prevent the tab from snagging and inadvertently separating from the tamper-evident closure 100. Also, the tab 168 being separated from the tamper-evident closure 100 creates a slight area of weakness and the tab ties 169 secure the tab 168 adding strength to the closure.

In this embodiment with the tear strip 164, the securing strap 170 overlies the tear strip 164. To remove the tear strip 164, the tab 168 is pulled to break the line of weakness 167 that connects the tear strip 164 to the second part 126, and then further, the tear strip 164 breaks the area of weakness 175 in the securing strap 170, as best shown in FIG. 6. Removal of the tear strip 164 removes the longitudinally extending protruding portion 165 to disengage the longitudinally extending protruding portion 165 from the longitudinally extending receiving portion 166. Therefore, when the tear strip 164 is used to open the tamper-evident closure 100, it disengages the locking connection between the first part 125 and the second part 126. Further, the tear strip 164 breaks the area of weakness 175 of the securing strap 170 irrevocably breaks the securing strap 170 so tampering is evident allowing the tamper-evident closure 100 to be opened without the need for a secondary tool.

It is understood the closure mechanism 160 could accomplish the same function in other ways, such as, for example, the second part 126 could include the longitudinally extending protruding portion 165 and the first part 125 could include the longitudinally extending receiving portion 166 that interlocks with the longitudinally extending protruding portion 165. Further alternative closure mechanism 160 could be used to secure the holding cavity closed, such as those described in PCT Publication WO 2021/024139.

In this embodiment with the securing strap 170 overlying a tear strip 164, the securing strap 170 can include a ramp edge 176, which align with the tear strip 164 (see FIG. 4). As the tear strip 164 is removed, the ramp edge 176 maintains alignment of the tear strip 164 with the area of weakness 175 so that the force applied by the tear strip 164 to the area of weakness 175 easily breaks the area of weakness 175. Without the ramp edge 176, the tear strip 164 might slide along the securing strap 170 and be misaligned with the area of weakness 175.

The tamper-evident closure 100 can include various access points 113. However, access points 113 can introduce points of egress into the holding cavity 110. For example, as show in FIG. 3, the tamper-evident closure 100 could include access points 113 on both opposing ends of the tamper-evident closure. But if an access point 113 is provided on both ends, then the medical access device 200 will include a cap 230 to prevent access into the medical access device 200. For accommodating various medical access devices 200, the tamper-evident closure 100 could include an access point 113 that is covered by an actuatable door 127. As shown in FIG. 2, the medical access device 200 only has a single tube 220, and in this embodiment the actuatable door 127 remains closed. As shown in FIG. 3, the medical access device 200 is a Y-site device with a side tube 220 and therefore the actuatable door is pressed inward into the holding cavity 110.

When the tamper-evident closure 100 includes an actuatable door 127, the second part 127 can include a door support wall 122. When the actuatable door 127 is not actuated, but left closing the potential access point (like FIG. 1 and 2), then the actuatable door 127 abuts the door support wall 122 preventing the actuatable door 127 from pressing inward and allowing egress into the holding cavity 110.

The tamper-evident closure 100 can include reinforcing ribs 121 to add structural strength to the holding cavity. As described, typically the tamper-evident closure 100 is length is greater than the width, this could cause weakness along the mid-section of the length. That weakness could allow a user to slightly squeeze and deform the tamper-evident closure 100. The deformation could disconnect the closure mechanism 160. Also, if an actuatable door 127 is included, the pressure to actuate the door 127 might cause the tamper-evident closure 100 to deform or flex too much making it difficult to actuate the door 127. Or if an actuatable door 127 is included, slight deformation might increase the space in and around the door 127 to allow a user to insert elongated objects into the holding cavity 110.

The securing strap 170 in this embodiment overlies the optional actuatable door 127. To allow for a potential tube 220 to exit from the access point 113 created when the actuatable door 127 is lowered, the securing strap 170 has a notch 177 through a portion aligned with the actuatable door 127. Then the securing strap 170 includes a partial opening to allow for the medical access device 200 to exit the tamper-evident closure 100 and therefore accommodate Y-site medical access devices 200.

In some embodiments, there is not an actuatable door 127, but instead the area where the actuatable door 127 is shown in these embodiments is an open access point 113. In those embodiments, the securing strap 170 itself could include at the notch 177 a removable portion functioning like the actuatable door that is filled in and covers the access point 113, or if removed provides an access point 113 for a tube 220.

In alternative designs of the tamper-evident closure containing a securing strap 170, the securing strap 170 itself serves as the primary closure holding the first part 125 together with the second part 126. In such an embodiment, the area of weakness 175 separates and then allow for the first part 125 and second part 126 to open and allow access into the holding cavity 110. In this type of construction the securing strap 170 could include a tab 168 to allow for easy, intentional separation of the first securing strap portion 172 from the second securing strap portion 175.

It is understood that both the first securing strap portion 172 and the second securing strap portion 174 could include one-way engaging fasteners such as described herein with the latch extension 171 and receiving latch 128a. It is understood that although the description of the securing strap 170 is with the second securing strap portion 174 securing to the second part 126, instead the securing strap 170 might instead have the first securing strap portion 172 securing to the first part 125 as described herein.

The securing strap 170 is shown aligned over the actuatable door 127. In other embodiments, the securing strap 170 can be along any portion of the tamper-evident closure 100, where the first securing strap portion 172 secures with the first part 125 and the second securing strap portion 174 secures to the second part. When the securing strap 170 is not over the door, then there is not a need to the notch 177. Typically, the securing strap 170 will span the entire height (z-direction) of the tamper evident closure 100.

In use, the medical access device 200 is placed in the open tamper-evident closure 100 such as shown in FIG. 3. The first part 125 and second part 126 close to form the holding cavity 110 around the medical access device 200, such as shown in FIG. 2. The free end 173 of the securing strap 170 is engaged with the second part 126 to hold the first part 125 and second part 126 closed. Specifically, the latch 171 locks in the slot 128. If included, the underlying closure mechanism 160, a tear strip 164 as shown, secures the first part 125 and second part 126 closed. In this embodiment the tear strip 164 aligns generally with the area of weakness 175 of the securing strip 170. The medical access device 200 is contained in the holding cavity 110 and access to the medical access device 200 is very limited.

To open the tamper-evident closure 100, the securing strap 170 is broken, which may separate the first part 125 from the second part 126. When there is a closure mechanism 160, such as the tear strip 164 shown, the tab 168 on the tear strip 164 is pulled to release the first part 125 from the second part 126, and the tear strip 164 also breaks the area of weakness 175 to separate the first securing strap portion 172 from the second securing strap portion 174.

To further aid in seeing any visual tampering, it could be that the securing strap 170 is one color and the tamper-evident closure 100 is a different color. Tamper-evident closures, such as those disclosed in this application, are designed to be closed and opened without the need for additional removal tools. For example, perforation lines, breakable securement straps, twistable or tearable structures or film can be used so that when a health care provider intentionally wants to access the vascular access device, it is easy to remove the tamper-evident closure. Also, it is desirable that when the tamper-evident closure is intentionally removed that the holding cavity opens cleanly with few separating parts.

The tamper-evident closure achieves the benefit of providing a strong holding cavity with limited ingress access into the holding cavity, that is easy to open without extra tools, and easy to break the securing strap.

Although specific embodiments have been shown and described herein, it is understood that these embodiments are merely illustrative of the many possible specific arrangements that can be devised in application of the principles of the invention.

## Claims

1. A tamper-evident closure (100) for a medical access device (200) for connecting to a patient's vascular system for administering medication or removing fluid, the tamper-evident closure comprising:
a first part (125) and a second part (126) forming a holding cavity (110), wherein the first part (125) is connected with the second part (126) by a hinge (123) such that actuation of the first part (125) relative to the second part (126) opens or closes access to the holding cavity (110);
an access point (113) into the holding cavity (110), wherein when the holding cavity (110) is open, the medical access device can be placed in the holding cavity, and wherein when the holding cavity (110) is closed, the medical access device (200) is secured within the holding cavity (110) and the access point allows a tube (220) of the medical access device (200) to exit the holding cavity (110);
a securing strap (170) for retaining the tamper-evident closure closed, wherein the securing strap (170) is provided to indicate if a user attempted to open the tamper-evident closure, the securing strap (170) comprising a first securing strap portion (172) integrally connected to the first part (125), a second securing strap portion (174) with a free end (173) for securing to the second part (126), and an area of weakness (175) separating the first securing strap portion (172) from the second securing strap portion (174), wherein the second securing strap portion (174) includes a latch extension (171) wherein the force to break the area of weakness (175) is less than the force to separate the securing strap (170) from the first part (125) or second part (126), **characterised in that** the second part (126) includes a slot (128) through the second part (126) and a receiving latch (128a) to receive the latch extension (171).

2. The tamper-evident closure of claim 1, wherein the first part (125) is pivotally connected to the second part (126) via the hinge (123).

3. The tamper-evident closure of anyone of the preceding claims, further comprising a closure mechanism, wherein the closure mechanism (160) comprises a tear strip (164) that interlocks the first part (125) with the second part (126) along a perimeter (111) of the holding cavity (110).

4. The tamper-evident closure of claim 3, wherein the closure mechanism separates from the tamper-evident closure to release the interlock of the first part (125) and second part (126).

5. The tamper-evident closure of claims 3 or 4, wherein the securing strap (170) includes a ramp edge (176) aligned to the tear strip.

6. The tamper-evident closure of claims 3, 4 or 5, wherein the area of weakness (175) of the securing strip overlies the tear strip (164).

7. The tamper-evident closure of any one of the preceding claims, wherein the area of weakness (175) is a series of perforations.

8. The tamper-evident closure of any one of the preceding claims, wherein the second securing strap portion (174) is irreversibly secured to the second part (126).

9. The tamper-evident closure of any one of the preceding claims, wherein the latch extension (171) has one way engagement with the receiving latch (128a).

10. The tamper-evident closure of any one of the preceding claims, further comprising a slot wall within the holding cavity (110) surrounding the latch extension (171).

11. The tamper-evident closure of claim 10, wherein the slot wall extends from the first part (125) to the second part (126) within the holding cavity (110).

12. The tamper-evident closure of any one of the preceding claims, wherein force to break the area of weakness (175) is less than the force to break the latch extension connection with the slot.

13. The tamper-evident closure of any one of the preceding claims, wherein the first part (125) includes an actuatable door for creating a second access point, optionally wherein the tamper-evident closure further comprises a door support wall extending from the second part (126) to engage with the actuatable door while the actuatable door is not actuated and is in connective engagement with the first part (125).

14. A method of enclosing a medical access device (200) into any one of the tamper-evident closures (100) of the preceding claims, comprising:
inserting the medical access device (200) into the holding cavity (110);
**characterised in that** the method further comprises the step of irreversibly securing the free end of the second securing strap portion (174) to the second part (126) via the latch extension (171) and the receiving latch (128a).

15. The method of claim 14, wherein removal of the closure mechanism breaks the securing strap (170).

## Patentansprüche

1. Manipulationsnachweisender Verschluss (100) für eine medizinische Zugangsvorrichtung (200) zum Verbinden mit dem Gefäßsystem eines Patienten zum Verabreichen von Medikamenten oder Entnehmen von Flüssigkeit, wobei der manipulationsnachweisende Verschluss aufweist:
einen ersten Teil (125) und einen zweiten Teil (126), die einen Haltehohlraum (110) ausbilden, wobei der erste Teil (125) mit dem zweiten Teil (126) durch ein Scharnier (123) derart verbunden ist, dass eine Betätigung des ersten Teils (125) relativ zu dem zweiten Teil (126) den Zugang zu dem Haltehohlraum (110) öffnet oder schließt;
einen Zugangspunkt (113) in den Haltehohlraum (110), wobei, wenn der Haltehohlraum (110) geöffnet ist, die medizinische Zugangsvorrichtung in dem Haltehohlraum platziert werden kann, und wobei, wenn der Haltehohlraum (110) geschlossen ist, die medizinische Zugangsvorrichtung (200) innerhalb des Haltehohlraums (110) gesichert ist und der Zugangspunkt es einem Schlauch (220) der medizinischen Zugangsvorrichtung (200) ermöglicht, aus dem Haltehohlraum (110) auszutreten;
ein Sicherungsband (170) zum Geschlossenhalten des manipulationsnachweisenden Verschlusses, wobei das Sicherungsband (170) bereitgestellt ist, um anzuzeigen, ob ein Benutzer versucht hat, den manipulationsnachweisenden Verschluss zu öffnen, das Sicherungsband (170) aufweisend einen ersten Sicherungsbandabschnitt (172), der mit dem ersten Teil (125) einstückig verbunden ist, einen zweiten Sicherungsbandabschnitt (174) mit einem freien Ende (173) zum Sichern an dem zweiten Teil (126) und einen Schwächungsbereich (175), der den ersten Sicherungsbandabschnitt (172) von dem zweiten Sicherungsbandabschnitt (174) trennt, wobei der zweite Sicherungsbandabschnitt (174) eine Riegelverlängerung (171) einschließt, wobei die Kraft, um den Schwächungsbereich (175) zu brechen, geringer ist als die Kraft, um das Sicherungsband (170) von dem ersten Teil (125) oder dem zweiten Teil (126) zu trennen, **dadurch gekennzeichnet, dass** der zweite Teil (126) einen Schlitz (128) durch den zweiten Teil (126) und einen Aufnahmeriegel (128a), um die Riegelverlängerung (171) aufzunehmen, einschließt.

2. Manipulationsnachweisender Verschluss nach Anspruch 1, wobei der erste Teil (125) über das Scharnier (123) mit dem zweiten Teil (126) schwenkbar verbunden ist.

3. Manipulationsnachweisender Verschluss nach einem der vorstehenden Ansprüche, ferner aufweisend einen Verschlussmechanismus, wobei der Verschlussmechanismus (160) einen Aufreißstreifen (164) aufweist, der den ersten Teil (125) mit dem zweiten Teil (126) entlang eines Umfangs (111) des Haltehohlraums (110) verriegelt.

4. Manipulationsnachweisender Verschluss nach Anspruch 3, wobei sich der Verschlussmechanismus von dem manipulationsnachweisenden Verschluss trennt, um die Verriegelung des ersten Teils (125) und des zweiten Teils (126) zu lösen.

5. Manipulationsnachweisender Verschluss nach Anspruch 3 oder 4, wobei das Sicherungsband (170) eine Rampenkante (176) einschließt, die auf den Aufreißstreifen ausgerichtet ist.

6. Manipulationsnachweisender Verschluss nach den Ansprüchen 3, 4 oder 5, wobei der Schwächungsbereich (175) des Sicherungsstreifens den Aufreißstreifen (164) überlagert.

7. Manipulationsnachweisender Verschluss nach einem der vorstehenden Ansprüche, wobei der Schwächungsbereich (175) eine Reihe von Perforationen ist.

8. Manipulationsnachweisender Verschluss nach einem der vorstehenden Ansprüche, wobei der zweite Sicherungsbandabschnitt (174) an dem zweiten Teil (126) irreversibel gesichert ist.

9. Manipulationsnachweisender Verschluss nach einem der vorstehenden Ansprüche, wobei die Riegelverlängerung (171) in Einwegeingriff mit dem Aufnahmeriegel (128a) steht.

10. Manipulationsnachweisender Verschluss nach einem der vorstehenden Ansprüche, ferner aufweisend eine Schlitzwand innerhalb des Haltehohlraums (110), die die Riegelverlängerung (171) umgibt.

11. Manipulationsnachweisender Verschluss nach Anspruch 10, wobei sich die Schlitzwand von dem ersten Teil (125) zu dem zweiten Teil (126) innerhalb des Haltehohlraums (110) erstreckt.

12. Manipulationsnachweisender Verschluss nach einem der vorstehenden Ansprüche, wobei die Kraft, um den Schwächungsbereich (175) zu brechen, geringer ist als die Kraft, um die Riegelverlängerungsverbindung mit dem Schlitz zu brechen.

13. Manipulationsnachweisender Verschluss nach einem der vorstehenden Ansprüche, wobei der erste Teil (125) eine betätigbare Tür zum Erzeugen eines zweiten Zugangspunkts einschließt, wobei optional der manipulationsnachweisende Verschluss ferner eine Türstützwand aufweist, die sich von dem zweiten Teil (126) erstreckt, um mit der betätigbaren Tür in Eingriff zu kommen, während die betätigbare Tür nicht betätigt ist und in verbindendem Eingriff mit dem ersten Teil (125) steht.

14. Ein Verfahren zum Aufnehmen einer medizinischen Zugangsvorrichtung (200) in einem der manipulationsnachweisenden Verschlüsse (100) der vorstehenden Ansprüche, aufweisend:
Einführen der medizinischen Zugangsvorrichtung (200) in den Haltehohlraum (110);
**dadurch gekennzeichnet, dass** das Verfahren ferner den Schritt eines irreversiblen Sicherns des freien Endes des zweiten Sicherungsbandabschnitts (174) an dem zweiten Teil (126) über die Riegelverlängerung (171) und den Aufnahmeriegel (128a) aufweist.

15. Das Verfahren nach Anspruch 14, wobei die Entfernung des Verschlussmechanismus ein Brechen des Sicherungsbands (170) bewirkt.

## Revendications

1. Fermeture inviolable (100) pour un dispositif d'accès médical (200) destiné à être relié au système vasculaire d'un patient pour administrer un médicament ou retirer un fluide, la fermeture inviolable comprenant :
une première partie (125) et une seconde partie (126) formant une cavité de maintien (110), dans laquelle la première partie (125) est reliée à la seconde partie (126) par une charnière (123) de telle sorte que l'actionnement de la première partie (125) par rapport à la seconde partie (126) ouvre ou ferme l'accès à la cavité de maintien (110) ;
un point d'accès (113) dans la cavité de maintien (110), dans laquelle lorsque la cavité de maintien (110) est ouverte, le dispositif d'accès médical peut être placé dans la cavité de maintien, et dans laquelle lorsque la cavité de maintien (110) est fermée, le dispositif d'accès médical (200) est fixé au sein de la cavité de maintien (110) et le point d'accès permet à un tube (220) du dispositif d'accès médical (200) de sortir de la cavité de maintien (110) ;
une sangle de fixation (170) destinée à maintenir la fermeture inviolable fermée, dans laquelle la sangle de fixation (170) est fournie pour indiquer si un utilisateur a tenté d'ouvrir la fermeture inviolable, la sangle de fixation (170) comprenant une première partie de sangle de fixation (172) intégralement reliée à la première partie (125), une seconde partie de sangle de fixation (174) avec une extrémité libre (173) destinée à être fixée à la seconde partie (126), et une zone de faiblesse (175) séparant la première partie de sangle de fixation (172) de la seconde partie de sangle de fixation (174), dans laquelle la seconde partie de sangle de fixation (174) comporte une extension de loquet (171), dans laquelle la force pour rompre la zone de faiblesse (175) est inférieure à la force pour séparer la sangle de fixation (170) de la première partie (125) ou de la seconde partie (126), **caractérisée en ce que** la seconde partie (126) comporte une fente (128) à travers la seconde partie (126) et un loquet de réception (128a) pour recevoir l'extension de loquet (171).

2. Fermeture inviolable selon la revendication 1, dans laquelle la première partie (125) est reliée de manière pivotante à la seconde partie (126) par l'intermédiaire de la charnière (123).

3. Fermeture inviolable selon l'une quelconque des revendications précédentes, comprenant en outre un mécanisme de fermeture, dans laquelle le mécanisme de fermeture (160) comprend une bande de déchirure (164) qui verrouille la première partie (125) avec la seconde partie (126) le long d'un périmètre (111) de la cavité de maintien (110).

4. Fermeture inviolable selon la revendication 3, dans laquelle le mécanisme de fermeture se sépare de la fermeture inviolable pour libérer le verrouillage de la première partie (125) et de la seconde partie (126).

5. Fermeture inviolable selon les revendications 3 ou 4, dans laquelle la sangle de fixation (170) comporte un bord de rampe (176) aligné sur la bande de déchirure.

6. Fermeture inviolable selon les revendications 3, 4 ou 5, dans laquelle la zone de faiblesse (175) de la bande de fixation recouvre la bande de déchirure (164).

7. Fermeture inviolable selon l'une quelconque des revendications précédentes, dans laquelle la zone de faiblesse (175) est une série de perforations.

8. Fermeture inviolable selon l'une quelconque des revendications précédentes, dans laquelle la seconde partie de sangle de fixation (174) est fixée de manière irréversible à la seconde partie (126).

9. Fermeture inviolable selon l'une quelconque des revendications précédentes, dans laquelle l'extension de loquet (171) vient en prise dans un seul sens avec le loquet de réception (128a).

10. Fermeture inviolable selon l'une quelconque des revendications précédentes, comprenant en outre une paroi de fente au sein de la cavité de maintien (110) entourant l'extension de loquet (171).

11. Fermeture inviolable selon la revendication 10, dans laquelle la paroi de fente s'étend de la première partie (125) à la seconde partie (126) au sein de la cavité de maintien (110).

12. Fermeture inviolable selon l'une quelconque des revendications précédentes, dans laquelle la force pour rompre la zone de faiblesse (175) est inférieure à la force pour rompre la liaison d'extension de loquet avec la fente.

13. Fermeture inviolable selon l'une quelconque des revendications précédentes, dans laquelle la première partie (125) comporte une porte actionnable destinée à créer un second point d'accès, facultativement dans laquelle la fermeture inviolable comprend en outre une paroi de support de porte s'étendant à partir de la seconde partie (126) pour venir en prise avec la porte actionnable alors que la porte actionnable n'est pas actionnée et est en prise de liaison avec la première partie (125).

14. Procédé d'enfermement d'un dispositif d'accès médical (200) dans l'une quelconque des fermetures inviolables (100) des revendications précédentes, comprenant :
l'insertion du dispositif d'accès médical (200) dans la cavité de maintien (110) ;
**caractérisé en ce que** le procédé comprend en outre l'étape consistant à fixer de manière irréversible l'extrémité libre de la seconde partie de sangle de fixation (174) à la seconde partie (126) par l'intermédiaire de l'extension de loquet (171) et du loquet de réception (128a).

15. Procédé selon la revendication 14, dans lequel le retrait du mécanisme de fermeture rompt la sangle de fixation (170).
